# EUROPEAN PATENT APPLICATION

(11) **EP 2 040 235 A1**
(43) Date of publication of application: **25.03.2009**
(21) Application number: 07018479.1
(22) Date of filing: 20.09.2007
(51) Int. Cl.: G08B 21/06, B60K 28/06, A61B 5/18

(54) **Apparatus for determining alertness of a driver steering a vehicle**

(71) Applicant: Al-Rasheed, Khaled M., 11543 Riyadh (SA)
(72) Inventor: Al-Rasheed, Khaled M., 11543 Riyadh (SA)
(74) Representative: Liesegang, Eva

(57) **Abstract**

An apparatus for determining a dangerous driving situation, in particular a lack of alertness of a driver of a vehicle, the vehicle having steering means, said apparatus comprising: a sensor for sensing a steering action carried out by a driver; means for deriving a steering pattern of a driver from the sensed steering action, the steering pattern including slopes where the steering angle is changed and pausing times during which no steering action is carried out; a memory for storing at least one of: a maximum normal slope gradient and a maximum normal pausing time of a steering pattern derived from the steering action of a given alert driver; a comparator for comparing at least one of: (i) the stored maximum slope gradient with a current slope gradient of a current steering pattern, and (ii) the stored maximum normal pausing time with a current pausing time of a current steering pattern of said driver and for generating an alarm signal when at least one of the current slope gradient exceeds the maximum normal slope gradient or the current pausing time exceeds the maximum normal pausing time; and warning means for alerting said driver in response to said alarm signal.

## Description

### Field of the invention

The invention relates to an apparatus for determining alertness of a driver steering a vehicle.

### Background of the Invention

US-A-5,465,079 describes a method and apparatus for evaluating a driver's performance under actual real-time conditions and for using such evaluation to determine the driver's ability to safely operate a vehicle. The method compares information gathered by a radar system and other sensors with information previously stored in an event recording device. Conditions monitored are used to make the determination as to whether the driver is performing in conformity with normal driving standards and the driver's own past performance. The system uses an event recording device that can personalize a vehicle to the driver associated with that event recording device. The system also monitors conditions external to a driver of a motor vehicle, such as the driving environment and time factors, such as time of day, trip length and duty day length. The profiles of a particular driver are gathered, these profiles including characterizations of the history of the throttle, speed, headway, steering, headlights, windshield wipers, and/or turn signal use. The steering profile is generated by monitoring the median frequency shifts, in other word, the variations in lane position. The frequency and amplitude of steering changes, correlated to the vehicle speed, provide a simplistic means for determining lane position. The lane position is considered to be an important profile in determining driver's fitness. If the driver's performance at any time during the trip is found to be below the personal standard calculated for the driver, the driver is alerted. Additionally, a dispatcher can be informed and the event can be recorded.

While the system of the US-A-5,465,079 can determine a driver's ability to safely operate a vehicle, it does so requiring a complex infrastructure including a radar system, a multiplicity of sensors, recording and communication means.

US 6 313 749 B1 discloses a system for detecting and monitoring lack of alertness of a driver. The system comprises a sensor for sensing a steering movement of a driver; a memory for storing a circadian rhythm pattern or time-of-day physiological reference profile of the predisposition to sleepiness; and means for computing steering transitions and weighing that computation according to time-of-day, to provide a warning indication of the driver's sleepiness. In other word, the system monitors steering performance of a driver, taking account of circadian and sleep parameters of an individual driver. For example, in exercising vehicle control, aberrant driver's steering behaviour, associated with degrees of driver's sleepiness, can be recognized and corrected or warning can be issued.

US 6,313,749 B1 describes that steering action or performance is best assessed when driving along a relatively straight road, when steering inputs of an alert driver are characterized by frequent, minor corrections. The patent shows examples for paired personal performance graphs reflecting steering wheel inputs for three individual drivers, each pair representing comparative alert and sleepy driving conditions, using an average value of steering wheel movement amplitude calculated by the RMS (root mean square) of the actual movements.

The system described in US 6,313,749 B1 is able to determine a driver's sleepiness, alertness of fitness. However, also this system is rather complex in terms of signals which are monitored and signal processing. The system is based on a correlation of driver control input and a circadian rhythm pattern which needs to be determined beforehand.

A similar system is described in DE 44 00 207 A1. According to this document, the heart beat of the driver and the steering angle are detected in order to determine alertness of the driver. This system appears to be disadvantageous in that it is necessary to monitor the driver's heart beat which is not practical in normal driving situations.

Another system for monitoring the driving conditions and issuing an alarm against unsafe driving behaviour is described in US-A-4,500,868.

In view of this prior art, it is an object of the present invention to provide an apparatus and a method for determining dangerous driving situations, in particular a lack of alertness of a driver of a vehicle, which are simple when compared to the prior art but still operate reliably.

### Summary of the Invention

This object is solved by an apparatus and a method for determining a dangerous driving situation, in particular a lack of alertness of a driver steering a vehicle, according to claims 1 and 5, respectively.

According to the present invention, a steering action carried out by the driver is sensed. From the sensed steering action, a steering pattern of the driver can be derived, the steering pattern including slopes where the steering angle is changed and pausing times during which no steering action is carried out. A memory is provided for storing at least one of a maximum normal slope gradient and a maximum normal pausing time of a steering pattern derived from the steering action of a given alert driver. That is, the system derives and stores the maximum slope gradient reflecting the speed of turning a steering wheel, or the maximum time during which no steering action takes place, or both, when a given alert driver is driving the vehicle. At least one of the stored maximum normal slope gradient is compared with a current slope gradient of a current steering pattern or the stored maximum normal pausing time is compared with a current pausing time of a current steering pattern and an alarm is generated if at least one of the current slope gradient exceeds the maximum normal slope gradient or the current pausing time exceeds the maximum normal pausing time in order to alert the driver.

The present invention is based on the perception that most traffic accidents caused by tired drivers or drivers just falling asleep take place in straight urban roads, highways and free ways (Autobahn). This is due to relatively boring driving conditions on such streets which do not require a lot of driving action, in particular when there is little traffic. Driving on such straight urban roads and highways is characterized by frequent, minor steering corrections in order to keep the vehicle in a straight parallel path within its respective driving lane.

The present invention establishes driving patterns for at least such driving situations, the driving patterns being characterized by pausing times during which no steering action is carried out and, more particularly, by a maximum normal pausing time. Pausing the steering wheel more than the maximum normal pausing time is considered to be a behaviour of a sleepy, non-alert driver.

The present invention aims at alerting drivers at the very beginning of the non-alert, sleep-like driving behaviour based on a very simple model which stores a maximum normal pausing time of a particular alert driver and continuously compares the current pausing times of said particular driver with the stored maximum normal pausing time. If the current pausing time exceeds the stored maximum normal pausing time, the present invention will alert at least the driver. Accordingly, the present invention provides a very simple and cheap system which can be easily manufactured, installed and used for alerting a driver when it is detected that the driver is not using the appropriate attention.

The present invention is also able to identify other dangerous driving situations, in particular those where a driver is making erratic and uncontrolled steering movements which might be caused by unfocussed driving behaviour of a distracted driver or even by a driver under the influence of alcohol or drugs. In such a case, it has been observed that steering movement is distinctively more abrupt generating steeper slopes, larger gradients, in the steering pattern. Accordingly, also the slope of the steering pattern can be used as a measure for identifying dangerous driving behaviour.

In one embodiment of the invention, the sensor for sensing a steering action can be a sensor mounted on or at a steering wheel or steering column of the vehicle. The sensor can be, but is not limited to, a resistive sensor, an electrical gyroscope, an acceleration sensor or any other type of a steering wheel movement sensor.

According to different embodiments of the invention, the warning means can be configures to generate an audio signal, a visual signal and/or a motion signal which is issued to the driver, passengers of the same vehicle, other drivers or third persons outside of the vehicle and/or to any remote preset destination.

According to a further aspect, the invention provides means for detecting the driving speed of the vehicle and for activating the apparatus only when the detected driving speed exceeds a predetermined threshold. It would be advantageous to activate the system of the present invention only above a certain driving speed in order to prevent disturbance while the system is not needed, e.g., when driving in a city at low speed. Preferably, the apparatus according to the invention is configured such that it activates itself as a function of the driving speed.

In addition to storing at least one of the maximum normal slope gradient and the maximum normal pausing time of a steering pattern derived from the steering action of a given alert driver, it can also be advantageous to store the complete steering pattern of the alert driver as well as the current steering pattern of the current driver in order to provide a full record of the driving performance.

Further, the apparatus and method according to the present invention can be used to determine the alertness of a number of drivers by providing and storing steering patterns and maximum normal slope gradients/pausing times of each of the drivers under alert driving condition. These stored steering patterns and maximum normal slope gradients/pausing times can then be used to be compared against the current steering patterns and slope gradients/pausing times of the respective drivers. At the beginning of each trip, a respective driver can select his/her profile which is to be compared against the current steering pattern.

In a preferred embodiment of the invention, the maximum normal slope gradient/pausing time is derived as a function of the driving speed and also the current slope gradient/pausing time is correlated with the current driving speed to be compared against the corresponding maximum normal slope gradients/pausing times.

The present invention also provides a system for determining alertness of a driver including a processor which is programmed with instructions for performing the method described above. The invention can further be embodied in a computer program including code for performing said method.

### Short Description of Drawings

Preferred embodiments of the invention are described with reference to the drawings in which:
- Fig. 1: shows an example of the steering action of a driver plotted versus time;
- Fig. 2: shows a plan view on a resistance ring used as a steering wheel sensor including a block diagram of an embodiment of the invention;
- Fig. 3: shows a side view of the steering wheel sensor attached to a steering column; and
- Fig. 4: shows a flow diagram of a first part of a preferred embodiment of the invention;
- Fig. 5: shows a block diagram of a second part of said preferred embodiment of the invention.
- Fig. 6: shows a flow diagram of a first part of another embodiment of the invention; and
- Fig. 7: shows a block diagram of a second part of said other embodiment of the invention.

### Detailed Description of Embodiments

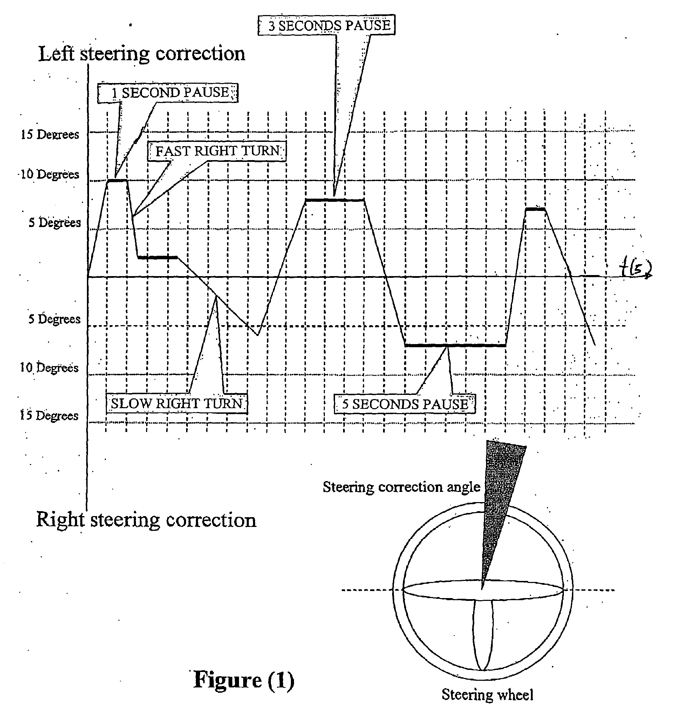

Fig. 1 schematically shows the steering action of a driver driving along a straight road, plotted versus time in units of seconds. When driving along a straight road, the driver will usually make minor left and right steering angle corrections in the range of about ±15 degrees relative to a reference position of the steering wheel, as schematically shown in the bottom right hand corner of Fig. 1.

In the graph of Fig. 1, pausing times are shown by horizontal bars whereas steering angle corrections are shown by sloped lines. In the exemplary graph of Fig. 1, the steering action starts with
- a left turn;
- followed by a pause of one second;
- followed by a fast right turn;
- followed by a pause of two seconds;
- followed by a slow right turn and a left turn;
- followed by a pause of three seconds;
- followed by a right turn;
- followed by a pause of five seconds;
- followed by a fast left turn;
- followed by a pause of one second; etc.

It should be noted that Fig. 1 is only used as an example for explaining the notion of the terms "steering wheel pattern", "slope gradient" and "pausing time" but it is not a realistic steering pattern where it is expected that steering angle corrections take place more frequently. Further, for establishing a useful steering pattern and a realistic maximum normal slope gradient and maximum normal pausing time, it will be necessary to determine a steering pattern of a driver for a greater length of time than shown in Fig. 1. Nevertheless, the graph of Fig. 1 is helpful for understanding the invention and, more particularly, the notions of "steering wheel pattern", "slope gradient" and "pausing time".

From Fig. 1, it can be recognized that the gradient of a slope, corresponding to a right turn or a left turn, becomes larger the faster the steering wheel is turned. Once the steering pattern has been determined, it can be analyzed to derive maximum slope gradients which occur under normal alert driving conditions ("maximum normal slope gradient"). Both the maximum normal slope gradient and the maximum normal pausing time preferably are correlated to predefined speed ranges. If, for a particular speed range, the slope gradient of the steering pattern exceeds a predetermined maximum normal slope gradient this will be considered as erratic uncontrolled dangerous driving behaviour.

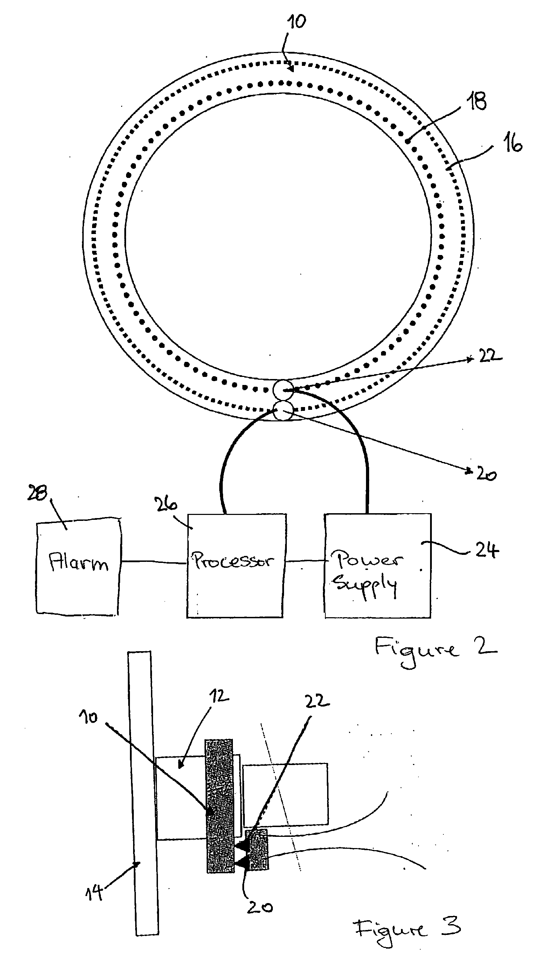

Figs. 2 and 3 show a plan view and a side view of one example of a steering wheel sensor 10 which is attached to a steering column 12 of a steering wheel 14. The steering wheel sensor 10 comprises a resistance ring including a resistive trace 16 and a power supply trace 18. The resistive trace 16 and the power supply trace 18 are contacted by a position detector 20 and a power supply contact 22, respectively. The position detector 20 slides along the resistive trace 18 and determines the rotational angle of the steering angle sensor, and thus of the steering wheel, by appropriately determining resistive changes along the circumference of the resistive trace 16. The resistive trace 16 is prepared such that its resistance changes in reproducible relation to the steering angle.

A power supply 24 provides power to the steering angle sensor 10 and a control unit or processor 26 adapted to process the signals received from the steering angle sensor 10. The processor 26 is representative of any suitable data processing means, including at least a memory and timer.

Accordingly, the steering angle sensor 10 is able to measure any steering angle corrections carried out by the driver. The processor 26 establishes a corresponding driving pattern for a driver and records this driving pattern as well as any maximum pausing time derived therefrom. Preferably, steering patterns are established in regard to preset driving speeds, such as 80 km per hour; 90 km per hour; 100 km per hour; etc. for a single driver.

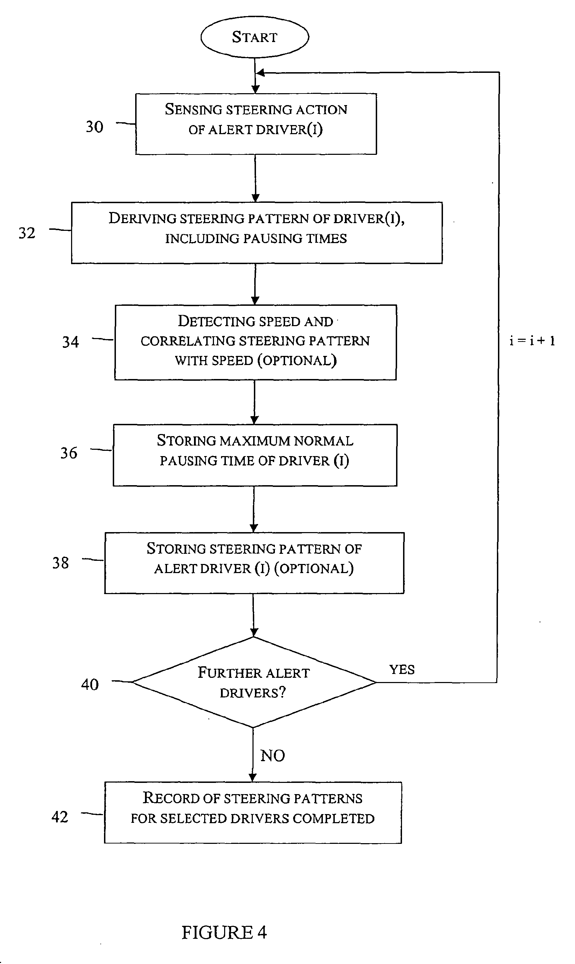

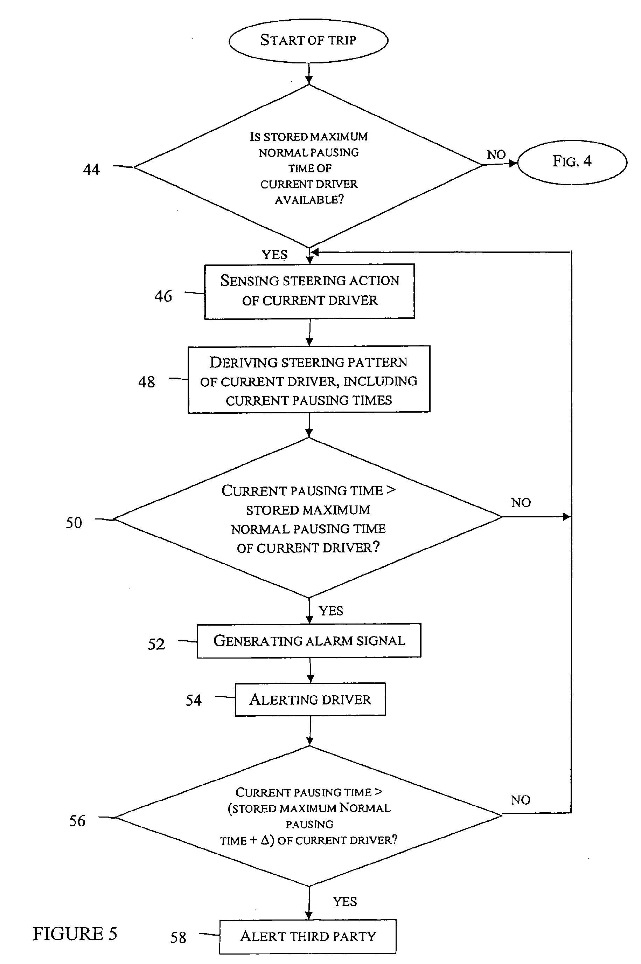

The steering angle sensor 10 and the processor 26 are used both for establishing one or more steering patterns of an alert driver as well as for monitoring current driver behaviour and comparing current driver behaviour to a stored steering pattern, as explained in further detail with reference to Figs. 4 and 5. The processor is further configured to issue an alarm signal to alarm means 28, as described in further detail with reference to Figs. 4 and 5. Instead of using the steering angle sensor 10 shown and described with respect to Figs. 2 and 3, an expert will easily implement alternative solutions, such as an electrical gyroscope, an acceleration sensor attached to the steering wheel or steering column, an encoder known from detecting the rotational position of an electric motor and the like. Further, the alarm means 28 can issue an optical, visual or motion signal wherein the alarm signal could be segmented or staged in the form of an increasing or decreasing warning. As mentioned above, the processor 26 is merely representative of any combination of electrical and computer devices useful for monitoring, recording, and comparing the steering action of a driver as well as activating different types of warning systems.

In the following, the present invention is explained with reference to an embodiment where only the pausing time of the steering pattern is considered in order to determine a dangerous driving situation, in particular a lack of alertness of a driver. However, the same principle can be used for evaluating the slope gradient of a steering pattern, alternatively or additionally to evaluating the pausing times, in order to determine a dangerous driving situation.

Fig. 3 shows a flow diagram of a first part of a method according to one embodiment of the invention. In this first part of the method, a record of steering patterns of individual drivers is established.

In a first step 30, this steering action of a first (i) alert driver is detected using a steering angle sensor. In a second step 32, a steering pattern of that particular driver is derived from the sensed steering action, the steering pattern including pausing times during which no steering action is carried out. Additionally, in a third step 34, the speed of the vehicle is detected and this speed is correlated with the steering pattern determined in step 32. The steering pattern comprises pausing times, as explained with reference to fig. 1, and more particularly, a maximal normal pausing time of the particular driver. In step 36, the maximum normal pausing time of the first driver (i) is stored. Additionally, the complete steering pattern of that particular driver can be stored, as shown in step 38.

Steps 30 to 38 are preferably carried out while a specific alert driver is driving on a straight road at predefined speed values in order to establish a full record of steering patterns and maximum pausing times of a particular driver when driving under alert conditions.

In this first part of the method according to the invention, shown in fig. 4, respective records of steering patterns and maximum pausing times can be established for more than one driver (i + 1, etc.). Therefore, in step 40, it is checked whether there are further drivers for which such a record shall be established. If yes, the method goes back to step 30; if no, the record of steering patterns for selected drivers is considered to be completed.

The record of steering patterns determined according to fig. 4 is used in the second part of the method of the present invention, shown in fig. 5. This second part of the method starts with the beginning of a trip of an individual driver whose driving performance shall be monitored. In a first step 44, it is determined whether a record of steering patterns for the current driver is available and, more particularly, if the maximum normal pausing time of the current driver has been stored in the system beforehand. If no, the second part of the method according to the invention can not be carried out, but rather the process shown in fig. 4 has to be carried out first. If yes, the method goes on to step 46 where the steering action of the current driver is determined, just in the same way as in step 30 of fig. 4.

From the steering action, the system derives a steering pattern of the current driver, including current pausing times, as shown in step 48. If the record of the stored steering pattern / maximum pausing time has been determined beforehand as a function of the driving speed, also step 48 will have to provide this correlation of steering pattern / pausing time and speed.

In step 50, it is determined whether the current pausing time exceeds the stored maximum normal pausing time determined for the current driver. If no, the method goes back to step 46. If yes, this determination is considered to be an indication of a lack of alertness of the driver who might be tired or possibly close to falling asleep. Therefore, in step 52, an alarm signal is generated which, in step 46, is used to alert the driver at the very beginning of his sleep-like driving behaviour. Alerting the driver can be done in different ways, such as by audio, visual and/or motion signal.

In the embodiment of fig. 5, it is also possible to alert a third party, such as a remote dispatcher, control station or the like. According to this embodiment, the third party is alerted, step 58, only if the current pausing time exceeds the stored maximum normal pausing time of the current driver plus a defined margin Δ, step 56. It is also feasible to alert a third party only if the driver him/herself already has been alerted a predefined number of times, indicating that the driver is ignoring respective warnings of the system.

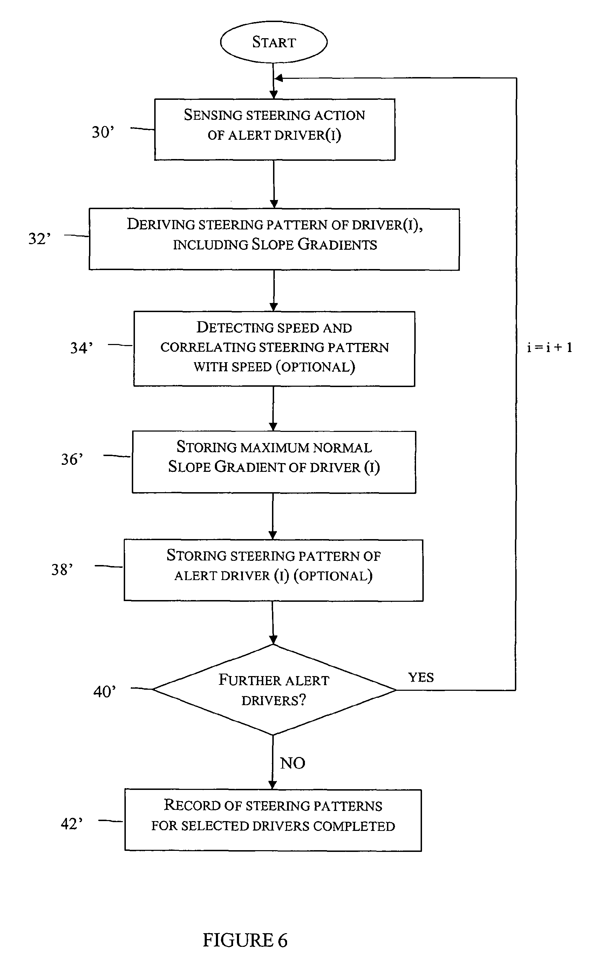

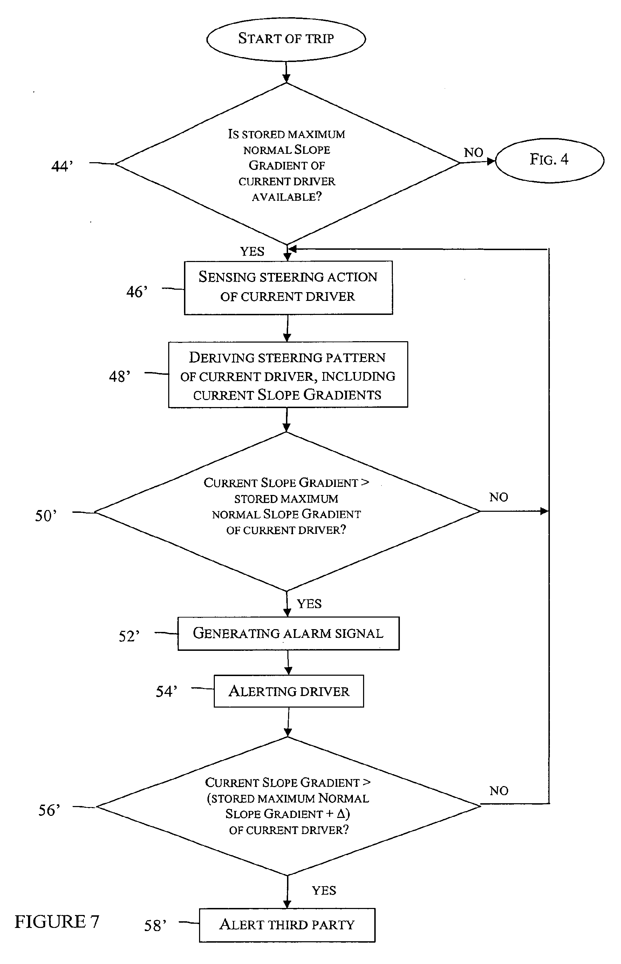

Figs. 6 and 7 show another embodiment of the method according to the present invention wherein the slope gradient of the steering pattern is used for determining dangerous driving behaviour, instead of the pausing times. Except for this difference, the embodiment of Figs. 6 and 7 fully corresponds to that of Figs. 4 and 5. The corresponding method steps are designnated by 30', 32', etc. Moreover, it is possible to monitor both pausing times and slope gradients of the steering pattern of a driver to determine dangerous driving behaviour and, particularly, a lack of alertness of a driver. Further, the skilled person may take into account other parameters of the steering pattern, alternatively or additionally to the parameters discussed above, in order to evaluate the driving behaviour of a driver steering a vehicle.

Many variations and modifications of the described system will be apparent to the skilled person. For example, the system could be programmed to active itself when driving above a preset speed in order not to cause disturbance while it is not needed, e.g. during low speed driving in a city environment. Signals can be communicated using wire-based or wireless communication technology. Further, all signals determined according to the invention, including the steering pattern, maximum normal pausing time, current pausing time, and alarm signal, can be recorded internally or externally to the system of the present invention.

## Claims

1. An apparatus for determining a dangerous driving situation, in particular a lack of alertness of a driver of a vehicle, the vehicle having steering means, said apparatus comprising:
a sensor for sensing a steering action carried out by a driver;
means for deriving a steering pattern of a driver from the sensed steering action, the steering pattern including slopes where the steering angle is changed and pausing times during which no steering action is carried out;
a memory for storing at least one of: a maximum normal slope gradient and a maximum normal pausing time of a steering pattern derived from the steering action of a given alert driver;
a comparator for comparing at least one of:
(i) the stored maximum slope gradient with a current slope gradient of a current steering pattern, and
(ii) the stored maximum normal pausing time with a current pausing time of a current steering pattern of said driver and
for generating an alarm signal when at least one of the current slope gradient exceeds the maximum normal slope gradient or the current pausing time exceeds the maximum normal pausing time; and
warning means for alerting said driver in response to said alarm signal.

2. The apparatus of claim 1 wherein the sensor is mounted on or at a steering wheel or steering column of the vehicle.

3. The apparatus of claim 1 or claim 2 wherein the warning means are configured to generate an audio signal, visual signal and/or motion signal.

4. The apparatus according to one of the preceding claims further comprising:
means for detecting the driving speed of the vehicle and for activating the apparatus when the detected driving speed exceeds a predetermined threshold.

5. A method for determining a dangerous driving situation, in particular a lack of alertness of a driver steering a vehicle, the method comprising the steps of:
(a) sensing a steering action carried out by a driver;
(b) deriving a steering pattern of a driver from the sensed steering action, the steering pattern including slopes where the steering angle is changed and pausing times during which no steering action is carried out;
(c) storing at least one of: a maximum normal slope gradient and a maximum normal pausing time of a steering pattern derived from the steering action of a first alert driver;
(d) comparing at least one of:
(i) the stored maximum normal slope gradient with a current slope gradient of a current steering pattern, and
(ii) the stored maximum normal pausing time with a current pausing time of a current steering pattern of said first driver;
(e) generating an alarm signal when at least one of the current slope gradient exceeds the maximum normal slope gradient or the current pausing time exceeds the maximum normal pausing time; and
(f) alerting said first driver in response to said alarm signal.

6. The method of claim 5 wherein said first driver is alerted using an audio signal, visual signal and/or motion signal.

7. The method of claim 5 or claim 6 comprising the additional step of:
alerting third persons within or outside of the vehicle in response to said alarm signal.

8. The method according to one of claims 5 to 7 comprising the additional step of:
detecting the driving speed of the vehicle and performing steps (d) to (f) when the detected driving speed exceeds a predetermined threshold.

9. The method according to one of claims 5 to 8 comprising the additional step of:
communicating the alarm signal to a remote location.

10. The method according to one of claims 5 to 9 comprising the additional step of:
recording steering patterns and at least one of slopes and pausing times of a steering pattern of a current driver.

11. The method according to one of claims 5 to 10 comprising the further steps of:
storing at least one of a maximum normal slope gradient and a maximum normal pausing time of a steering pattern derived from the steering action of a second alert driver; comparing at least one of:
(i) the stored maximum normal slope gradient of said second driver with a current slope gradient of a current steering pattern of said second driver, and
(ii) the stored maximum normal pausing time of said second driver with a current pausing time of a current steering pattern of said second driver; and
generating an alarm signal when at least one of the current slope gradient exceeds the maximum slope gradient or the current pausing time exceeds the maximum normal pausing time; and
alerting said second driver in response to said alarm signal.

12. The method of one of claims 5 to 11, wherein the maximum normal slope gradient and /or the maximum normal pausing time is derived as a function of the driving speed.

13. The method of one of claims 5 to 12, wherein individual steering patterns are established and stored for individual drivers.

14. A system for determining alertness of a driver comprising:
a sensor for sensing a steering action of the driver; and
a processor including instructions for performing the method of one of claims 5 to 13.
